Europäisches Patentamt
European Patent Office
Office européen des brevets

(11) Veröffentlichungsnummer: **0 200 118**
**A2**

(12) # EUROPÄISCHE PATENTANMELDUNG

(21) Anmeldenummer: 86105396.5

(22) Anmeldetag: 18.04.86

(51) Int. Cl.[4]: C 07 D 249/08
C 07 D 233/60, A 01 N 43/653
A 01 N 43/50

(30) Priorität: 30.04.85 DE 3515503

(43) Veröffentlichungstag der Anmeldung:
05.11.86 Patentblatt 86/45

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Kraatz, Udo, Dr.
Andreasstrasse 22a
D-5090 Leverkusen(DE)

(72) Erfinder: Büchel, Karl Heinz, Prof. Dr.
Dabringhausener Strasse 42
D-5093 Burscheid(DE)

(72) Erfinder: Hänssler, Gerd, Dr.
Am Arenzberg 58a
D-5090 Leverkusen 3(DE)

(72) Erfinder: Reinecke, Paul, Dr.
Steinstrasse 8
D-5090 Leverkusen 3(DE)

(54) **Substituierte Azolylmethylether.**

(57) Neue substituierte Azolylmethylether der Formel

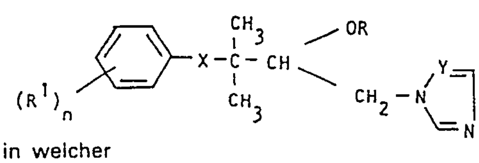

(I)

in welcher

R für Alkyl, Alkenyl, Halogenalkenyl, Alkinyl, Cycloalkyl oder für gegebenenfalls substituiertes Aralkyl steht,

$R^1$ für Halogen, Alkyl, Alkoxy, Alkylthio, Alkylsulfonyl, Halogenalkyl, Halogenalkoxy, Nitro, Cyano, Alkoxycarbonyl oder gegebenenfalls substituiertes Phenyl steht,

X für Sauerstoff oder Schwefel steht,

Y für Stickstoff oder CH steht und

n für die Zahlen 0, 1, 2 oder 3 steht,

sowie deren Säureadditions-Salze und Metallsalz-Komplexe, ein Verfahren zur Herstellung der neuen Stoffe und deren Verwendung als Fungizide.

EP 0 200 118 A2

86105396.5

0200118



BAYER AKTIENGESELLSCHAFT 5090 Leverkusen, Bayerwerk
Konzernverwaltung RP
Patentabteilung Dü/Kü-c
Ia

## Substituierte Azolylmethylether

Die Erfindung betrifft neue Azolylmethylether, ein Verfahren zu ihrer Herstellung und ihre Verwendung als Fungizide.

Es ist bereits bekannt geworden, daß zahlreiche Azolylmethylether zur Bekämpfung von Pilzen geeignet sind (vgl. DE-OS 2 547 953 und DE-OS 2 653 420). Die Wirksamkeit dieser Stoffe ist jedoch, vor allem bei niedrigen Aufwandmengen, nicht immer voll befriedigend.

Es wurden nun neue substituierte Azolylmethylether der Formel

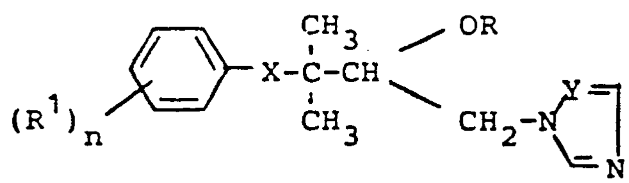

(I)

in welcher

R für Alkyl, Alkenyl, Halogenalkenyl, Alkinyl, Cycloalkyl oder für gegebenenfalls substituiertes Aralkyl steht,

$R^1$ für Halogen, Alkyl, Alkoxy, Alkylthio, Alkylsulfonyl, Halogenalkyl, Halogenalkoxy, Nitro, Cyano, Alkoxycarbonyl oder gegebenenfalls substituiertes Phenyl steht,

X für Sauerstoff oder Schwefel steht,

Y für Stickstoff oder CH steht und

n für die Zahlen 0, 1, 2 oder 3 steht,

sowie deren Säureadditions-Salze und Metallsalz-Komplexe gefunden.

Die erfindungsgemäßen Stoffe enthalten ein asymmetrisch substituiertes Kohlenstoffatom. Sie können daher in Form von Racematen oder auch in Form von optisch aktiven Enantiomeren vorliegen. Die Erfindung betrifft sowohl Racemate als auch optisch aktive Enantiomere.

Weiterhin wurde gefunden, daß man substituierte Azolylmethylether der Formel (I) sowie deren Säureadditionssalze und Metallsalz-Komplexe erhält, wenn man Azolylmethylcarbinole der Formel

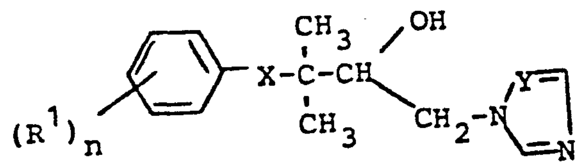

(II)

in welcher

$R^1$, X, Y und n die oben angegebene Bedeutung haben,

mit Verbindungen der Formel

R-Z (III)

in welcher

R die oben angegebene Bedeutung hat

und

Z für Halogen, Tosyl, Mesyl, Sulfat oder eine leicht abspaltbare Säuregruppe steht,

in Gegenwart eines Säurebindemittels sowie in Gegenwart eines Verdünnungsmittels und gegebenenfalls in Gegenwart eines Katalysators umsetzt und gegebenenfalls an die so

erhaltenen Verbindungen der Formel (I) eine Säure oder ein Metallsalz addiert.

Schließlich wurde gefunden, daß die neuen substituierten Azolylmethylether der Formel (I) sowie deren Säureadditionssalze und Metallsalz-Komplexe starke fungizide Eigenschaften besitzen.

Es ist als äußerst überraschend zu bezeichnen, daß die erfindungsgemäßen Stoffe eine deutlich bessere fungizide Wirksamkeit aufweisen als die konstitutionell ähnlichsten, aus dem Stand der Technik vorbekannten Verbindungen gleicher Wirkungsrichtung.

Die erfindungsgemäßen substituierten Azolylmethylether sind durch die Formel (I) allgemein definiert. In dieser Formel steht

R vorzugsweise für geradkettiges oder verzweigtes Alkyl mit 1 bis 6 Kohlenstoffatomen, Alkenyl mit 2 bis 6 Kohlenstoffatomen, Halogenalkenyl mit 2 bis 6 Kohlenstoffatomen und 1 bis 5 Halogenatomen, Alkinyl mit 2 bis 6 Kohlenstoffatomen, Cycloalkyl mit 3 bis 8 Kohlenstoffatomen oder für gegebenenfalls durch Halogen und/oder Alkyl mit 1 bis 4 Kohlenstoffatomen substituiertes Aralkyl mit 6 bis 10 Kohlenstoffatomen im Arylteil und 1 bis 4 Kohlenstoffatomen im Alkylteil,

$R^1$ vorzugsweise für Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil sowie für gegebenenfalls durch Halogen und/oder Alkyl mit 1 bis 4 Kohlenstoffatomen substituiertes Phenyl,

X für Sauerstoff oder Schwefel,

Y für Stickstoff oder CH und

n für die Zahlen 0, 1, 2 oder 3.

Besonders bevorzugt sind diejenigen substituierten Azolylmethylether der Formel (I), in denen

R für geradkettiges oder verzweigtes Alkyl mit 1 bis 4 Kohlenstoffatomen, Alkenyl mit 2 bis 5 Kohlenstoffatomen, Halogenalkenyl mit 2 bis 4 Kohlenstoffatomen und 1 bis 3 Chloratomen, Alkinyl mit 2 bis 5 Kohlenstoffatomen, Cycloalkyl mit 3 bis 7 Kohlenstoffatomen oder für gegebenenfalls durch Fluor, Chlor, Brom und/oder Alkyl mit 1 bis 3 Kohlenstoffatomen substituiertes Phenylalkyl mit 1 oder 2 Kohlenstoffatomen im Alkylteil steht,

$R^1$ für Fluor, Chlor, Brom, Methyl, Ethyl, Isopropyl, tert.-Butyl, Methoxy, Methylthio, Ethoxy, Ethylthio, Trifluormethyl, Nitro, Cyano, Trifluormethoxy, Methoxycarbonyl, Ethoxycarbonyl sowie für gegebenenfalls ein- bis dreifach, gleichartig oder verschieden durch Fluor, Chlor, Methyl und/oder Ethyl substiutiertes Phenyl steht,

X für Sauerstoff oder Schwefel steht,

Y für Stickstoff oder CH steht und

n für die Zahlen 0, 1, 2 oder 3 steht.

Bevorzugte erfindungsgemäße Verbindungen sind auch Additionsprodukte aus Säuren und denjenigen substituierten Azolylmethylethern der Formel (I), in denen R, $R^1$, X, Y und n die Bedeutungen haben, die bereits vorzugsweise für diese Reste bzw. diesen Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen substituierten Azolylmethylethern der Formel (I), in denen R, $R^1$, X, Y und n die Bedeutungen haben, die bereits vorzugsweise für diese Reste bzw. diesen Index genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man 3-(2,4-Dichlor-phenoxy)-3-methyl-1-(1,2,4-triazol-1-yl)-butan-2-ol und 4-Chlor-benzylchlorid als Ausgangsstoffe, so kann der Verlauf des erfindungsgemäßen Verfahrens durch das folgende Formelschema wiedergegeben werden.

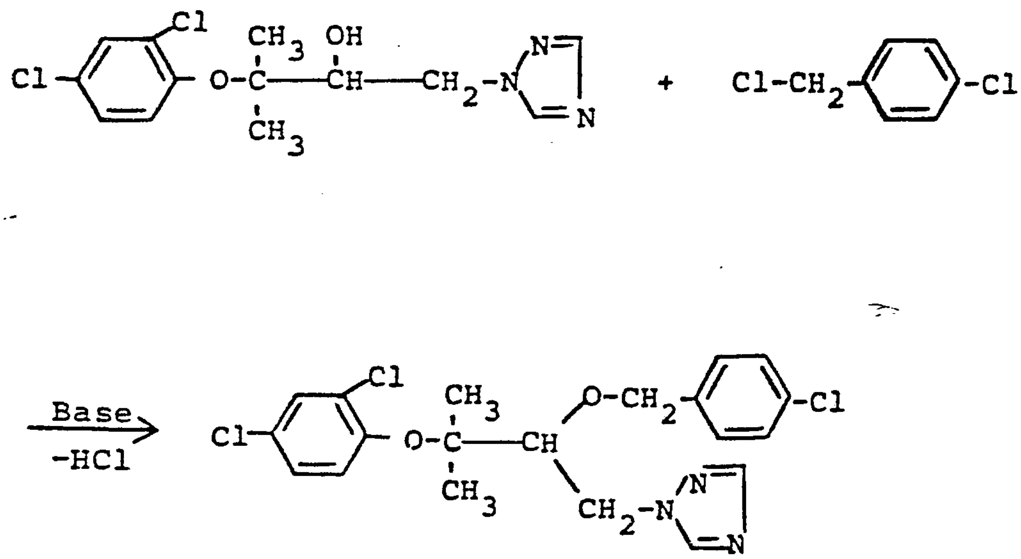


Die bei der Durchführung des erfindungsgemäßen Verfahrens als Ausgangsstoffe benötigten Azolylmethylcarbinole sind durch die Formel (II) allgemein definiert. In dieser Formel haben $R^1$, X, Y und n vorzugsweise diejenigen Bedeutungen, die bereits im Zusammenhang mit der Beschreibung der erfindungsgemäßen Stoffe der Formel (I) vorzugsweise für diese Substituenten bzw. diesen Index genannt wurden.

Die Azolylmethylcarbinole der Formel (II) sind bekannt (vgl. DE-OS 3 048 267). Man erhält Azolylmethylcarbinole der Formel (II), indem man Halogenketone der Formel

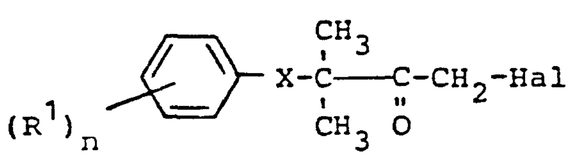

(IV)

in welcher

$R^1$, X und n die oben angegebene Bedeutung haben und

Hal für Chlor oder Brom steht,

zunächst mit einem komplexen Hydrid, wie Natriumboranat, in Gegenwart eines Verdünnungsmittels, wie z.B. Methanol, Ethanol oder Tetrahydrofuran, bei Temperaturen zwischen 20 und 80°C umsetzt und die dabei entstehenden Stoffe der Formel

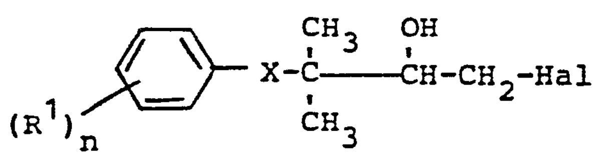

(V)

in welcher

$R^1$, X, Hal und n die oben angegebene Bedeutung haben,

anschließend mit einem Azol der Formel

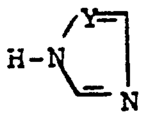

(VI)

in welcher

Y die oben angegebene Bedeutung hat,

in Gegenwart einer Base, wie z.B. Kaliumcarbonat, sowie in Gegenwart eines Verdünnungsmittels, wie z.B. Aceton, Acetonitril oder Dioxan, bei Temperaturen zwischen 20 und 100°C umsetzt.

Die bei dem erfindungsgemäßen Verfahren weiterhin als Ausgangsstoffe benötigten Verbindungen sind durch die Formel (III) definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Substituenten genannt wurden. Z steht vorzugsweise für Chlor, Brom, Iod, Tosyl, Mesyl und Sulfat.

Die Verbindungen der Formel (III) sind bereits bekannt oder lassen sich nach im Prinzip bekannten Verfahren in einfacher Weise herstellen.

Als Verdünnungsmittel kommen bei der Durchführung des erfindungsgemäßen Verfahrens organische Solventien in Frage. Vorzugsweise verwendbar sind Ketone, wie Diethylketon, Methylethylketon und Aceton, ferner Nitrile, wie Propionitril und Acetonitril, außerdem Alkohole, wie Ethanol und Isopropanol, weiterhin Ether, wie Tetrahyrofuran und Dioxan, darüberhinaus Kohlenwasserstoffe, wie Benzol und Toluol, und ferner stark polare Solventien, wie Dimethylformamid, Dimethylsulfoxid und Sulfolan.

Als Säurebindemittel kommen bei der Durchführung des erfindungsgemäßen Verfahrens alle üblicherweise verwendbaren anorganischen und organischen Basen in Betracht. Vorzugsweise einsetzbar sind Alkalicarbonate, wie Natriumcarbonat, Kaliumcarbonat und Natriumhydrogencarbonat, Alkalihydroxide, wie Natriumhydroxid oder Kaliumhydroxid, Alkalihydride wie Natriumhydrid, Alkalialkoholate, wie Natrium-methylat, Natrium-ethylat oder Kalium-tert.-butylat, sowie niedere tertiäre Alkylamine, Cycloalkylamine und Aralkylamine, wie Triethylamin, N,N-Dimethylcyclohexylamin, Dicyclohexylamin, N,N-Dimethylbenzylamin und Diazabicyclooctan, sowie aromatische Amine, wie z.B. Pyridin.

Als Katalysatoren können bei der Durchführung des erfindungsgemäßen Verfahrens alle für derartige Umsetzungen üblichen Reaktionsbeschleuniger eingesetzt werden. Vorzugsweise verwendbar sind Alkylammonium-Salze, wie Trimethyl-benzylammoniumhydroxid ("Triton B"), Triethyl-benzylammoniumchlorid, Benzyl-dodecyl-dimethylammoniumchlorid ("Zephirol"), Tetrabutylammoniumbromid und Methyl-trioctyl-ammoniumchlorid.

Die Reaktionstemperaturen können bei der Durchführung des erfindungsgemäßen Verfahrens in einem größeren Bereich variiert werden. Im allgemeinen arbeitet man bei Temperaturen zwischen 20 und 150° C, vorzugsweise zwischen 30 und 100° C.

Bei der Durchführung des erfindungsgemäßen Verfahrens arbeitet man im allgemeinen unter Normaldruck. Es ist jedoch auch möglich, unter erhöhtem oder vermindertem Druck zu arbeiten.

Bei der Durchführung des erfindungsgemäßen Verfahrens setzt man auf 1 Mol Azolylmethylcarbinol der Formel (II) vorzugsweise 1 bis 1,5 Mol an einer Verbindung der Formel (III) und 1 bis 2 Mol an Säurebindemittel sowie gegebenenfalls 0,05 bis 0,2 Mol eines Katalysators ein. Die Isolierung der erfindungsgemäßen Stoffe erfolgt nach üblichen Methoden. Im allgemeinen verfährt man in der Weise, daß man das Reaktionsgemisch unter vermindertem Druck einengt, mit Wasser und einem mit Wasser wenig mischbaren organischen Solvens versetzt, die organische Phase abtrennt, mit Wasser wäscht, trocknet und einengt. Es ist jedoch auch möglich, das Reaktionsgemisch mit Wasser zu versetzen, das fest anfallende Produkt abzusaugen, zu waschen und zu trocknen.

Zur Herstellung von Säureadditions-Salzen der Verbindungen der Formel (I) kommen alle diejenigen Säuren in Frage, die zu physiologisch verträglichen Salzen führen. Vorzugsweise verwendbar sind diejenigen Säuren, die bereits im Zusammenhang mit der Beschreibung der erfindungsgemäßen Stoffe als bevorzugt zu addierende Säuren genannt wurden.

Die Säureadditions-Salze der Verbindungen der Formel (I) lassen sich in einfacher Weise nach üblichen Salzbildungsmethoden herstellen. Im allgemeinen verfährt man so, daß man eine Verbindung der Formel (I) in einem geeigneten inerten Verdünnungsmittel löst und dann eine Säure hinzufügt. Die Isolierung erfolgt in bekannter Weise, zum Beispiel dadurch, daß man das Salz abfiltriert und gegebenenfalls durch Waschen mit einem inerten organischen Lösungsmittel reinigt.

Zur Herstellung von Metallsalz-Komplexen der Verbindungen der Formel (I) kommen vorzugsweise Salze von denjenigen Metallen in Betracht, die bereits im Zusammenhang mit der Beschreibung der erfindungsgemäßen Stoffe als bevorzugt zu addierende Metallsalze genannt wurden. Als Anionen dieser Metallsalze kommen vorzugsweise Halogenwasserstoffsäuren, wie zum Beispiel Chlorwasserstoffsäure und Bromwasserstoffsäure, ferner Phosphorsäure, Salpetersäure und Schwefelsäure in Frage.

Die Metallsalz-Komplexe von Verbindungen der Formel (I) lassen sich in einfacher Weise nach üblichen Methoden herstellen. Im allgemeinen geht man so vor, daß man ein

Metallsalz in Alkohol, wie zum Beispiel Ethanol, löst und dann eine Verbindung der Formel (I) hinzufügt. Die Isolierung erfolgt ebenfalls in bekannter Weise, zum Beispiel dadurch, daß man den Metallsalz-Komplex abfiltriert und gegebenenfalls durch Umkristallisation reinigt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;

Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Cercospora-Arten, wie Cercospora canescens, Botrytis-Arten, wie Botrytis cinerea, Pellicularia-Arten, wie Pellicularia sasakii an Reis, Pyricularia-Arten, wie Pyricularia oryzae an Reis, und ferner zur Bekämpfung von Erregern an Getreidepflanzen, wie z.B. Erysiphe graminis, Cochliobolus sativus, Pyrenophora teres, Leptosphaeria nodorum und Drechslera graminea sowie Fusarium-Arten verwenden. Die erfindungsgemäßen Wirkstoffe besitzen auch eine sehr gute bakterizide Wirkung, z.B. gegen Xanthomonas oryzae an Reis.

Im übrigen besitzen die erfindungsgemäßen Wirkstoffe auch pflanzenwuchsregulierende Eigenschaften.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder

Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten, oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B.

nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß,

Wuchsstoffen, Pflanzennährstoffen und Bodenstrukturverbesserungsmitteln.

Die Wirkstoffe können als solche, in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei der Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die Herstellung und die Verwendung der erfindungsgemäßen Wirkstoffe geht aus den folgenden Beispielen hervor.

## Herstellungsbeispiele

### Beispiel 1

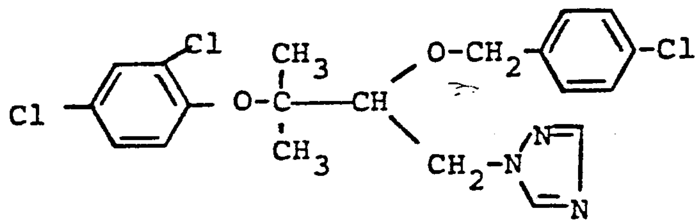

In eine Lösung von 31,6 g (0,1 Mol) 3-Methyl-3-(2,4-dichlor-phenoxy)-1-(1,2,4-triazol-1-yl)-butan-2-ol in 150 ml Dimethylsulfoxid werden 10 ml einer 50 %igen wäßrigen Benzyl-dodecyl-dimethylammoniumchlorid-Lösung und 6 g (0,15 Mol) Natriumhydroxid-Pulver gegeben. Danach werden unter Rühren bei 20°C auf einmal 19,3 g (0,12 Mol) 4-Chlorbenzylchlorid hinzugefügt. Hierbei tritt eine leicht exotherme Reaktion ein, wobei die Temperatur des Reaktionsgemisches auf 45°C ansteigt. Nach dem Abklingen der Reaktion läßt man noch 30 Minuten nachrühren und gießt dann das Reaktionsgemisch in 1 Liter Wasser. Das ausfallende Produkt wird abgesaugt, nacheinander mit Wasser und Petrolether gewaschen und bei 80°C unter vermindertem Druck getrocknet. Man erhält auf diese Weise 34 g (77 % der Theorie) an 2-(2,4-Dichlorphenoxy)-2-methyl-3-(4-chlor-benzyloxy)-4-(1,2,4-triazol-1-yl)-butan in Form eines kristallinen Produktes vom Schmelzpunkt 108°C.

Beispiel 2

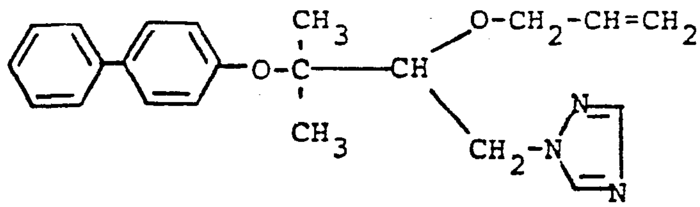

Eine Lösung von 18 g (0,06 Mol) 3-Methyl-3-(4-phenyl-phenoxy)-1-(1,2,4-triazol-1-yl)-butan-2-ol in 150 ml Dimethylformamid wird unter Eiskühlung mit 3,6 g (0,12 Mol) 80 %igem Natriumhydrid versetzt. Nach dreißigminütigem Rühren bei 20°C werden 14,5 g (0,12 Mol) 3-Brompropen in das Reaktionsgemisch eingetropft. Dabei erfolgt eine leicht exotherme Reaktion, wodurch die Temperatur des Reaktionsgemisches auf 45°C ansteigt. Nach dem Abklingen der Reaktion rührt man noch 30 Minuten bei 20°C und gießt das Reaktionsgemisch dann in 1 Liter Wasser. Das ausfallende Produkt wird abgesaugt und nach dem Waschen mit Wasser unter vermindertem Druck getrocknet. Man erhält auf diese Weise 16 g (74 % der Theorie) an 2-(4-Phenyl-phenoxy)-2-methyl-3-(prop-2-en-1-oxy)-4-(1,2,4-triazol-1-yl)-butan in Form eines kristallinen Produktes vom Schmelzpunkt 51-52°C.

Nach den in den Beispielen 1 und 2 angegebenen Methoden werden auch die in der folgenden Tabelle formelmäßig aufgeführten Stoffe der Formel (I) hergestellt.

Tabelle 1

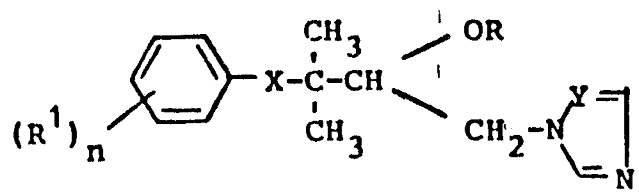

(I)

| Beispiel Nr. | $(R^1)_n$ | X | Y | R | Schmelzpunkt [°C] |
|---|---|---|---|---|---|
| 3 | 4-Cl | O | N | $-CH_2-CH=CH_2$ | 50 |
| 4 | 4-Cl | O | N | $-CH_2-C\equiv CH$ | 85 |
| 5 | 4-Cl | O | CH | $-CH_2-$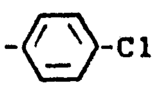$-Cl$ | 108 |
| 6 | 2,4-$Cl_2$ | O | N | $-CH_2-CH=CH_2$ | Öl |
| 7 | 2,4-$Cl_2$ | O | N | $-CH_2-C\equiv CH$ | Harz |

Tabelle 1 (Fortsetzung)

| Beispiel Nr. | $(R^1)_n$ | X | Y | R | Schmelzpunkt [°C] |
|---|---|---|---|---|---|
| 8 | 4-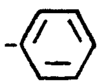 | O | N | $-CH_2-C\equiv CH$ | 119-121 |
| 9 | 4-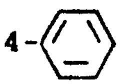 | O | N | $-CH_2-$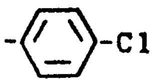$-Cl$ | 130 |
| 10 | 4-Cl, 2-$CH_3$ | O | N | $-CH_2-CH=CH_2$ | Harz |
| 11 | 4-Cl, 2-$CH_3$ | O | N | $-CH_2-C\equiv CH$ | 78 |
| 12 | 4-Cl, 2-$CH_3$ | O | N | $-CH_2-$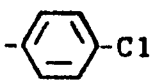$-Cl$ | 68-70 |
| 13 | 2,4-$Cl_2$ | O | N | $-CH_2-C(Cl)=CH_2$ | Harz |
| 14 | 2,4-$Cl_2$ | O | CH | $-CH_2-CH=CH_2$ | Harz |
| 15 | 2,4-$Cl_2$ | O | CH | $-CH_2-C\equiv CH$ | Harz |
| 16 | 2,4-$Cl_2$ | O | CH | $-CH_2-$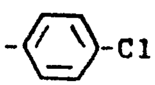$-Cl$ | 70 |

Tabelle 1 (Fortsetzung)

| Beispiel Nr. | $(R^1)_n$ | X | Y | R | Schmelzpunkt [°C] |
|---|---|---|---|---|---|
| 17 | 4-Cl | S | N | $-CH_2-C\equiv CH$ | Harz |
| 18 | 4-Cl | S | N | $-CH_2-$ 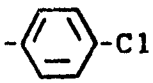 -Cl | Harz |
| 19 | 2-Cl | O | N | $-CH_2-CH=CH_2$ | Harz |
| 20 | 2-Cl | O | N | $-CH_2-C\equiv CH$ | Harz |
| 21 | 2-Cl | O | N | $-CH_2-$ 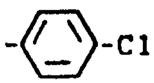 -Cl | 55 |
| 22 | 2-Cl | O | CH | $-CH_2-CH=CH_2$ | Harz |
| 23 | 2-Cl | O | CH | $-CH_2-C\equiv CH$ | Harz |
| 24 | 2-Cl | O | CH | $-CH_2-$ 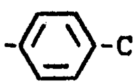 -Cl | Harz |
| 25 | 4-Cl, 3-$CF_3$ | S | N | $-CH_2-CH=CH_2$ | Harz |

Tabelle 1 (Fortsetzung)

| Beispiel Nr. | $(R^1)_n$ | X | Y | R | Schmelzpunkt [°C] |
|---|---|---|---|---|---|
| 26 | 4-Cl, 3-$CF_3$ | S | N | $-CH_2-C{\equiv}CH$ | Harz |
| 27 | 3,4-$Cl_2$ | O | CH | $-CH_2-CH{=}CH_2$ | Harz |
| 28 | 3,4-$Cl_2$ | O | CH | $-CH_2-C{\equiv}CH$ | Harz |
| 29 | 3,4-$Cl_2$ | O | CH | 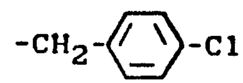 | Harz |
| 30 | 3,4-$Cl_2$ | O | N | $-CH_2-CH{=}CH_2$ | Harz |
| 31 | 3,4-$Cl_2$ | O | N | $-CH_2-C{\equiv}CH$ | 85 |
| 32 | 3,4-$Cl_2$ | O | N | 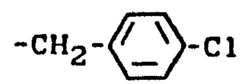 | Harz |
| 33 | 3,4-$Cl_2$ | O | N | 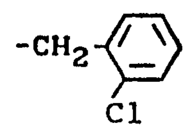 | 46-48 |

0200118

Le A 23 685

Tabelle 1 (Fortsetzung)

| Beispiel Nr. | $(R^1)_n$ | X | Y | R | Schmelzpunkt [°C] |
|---|---|---|---|---|---|
| 34 | 3,4-$Cl_2$ | O | N | -$CH_2$- 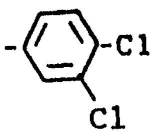 | Harz |
| 35 | 3,4-$Cl_2$ | O | N | -$CH_2$- 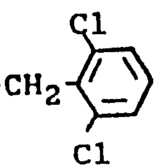 | Harz |
| 36 | 4-Cl, 2-$CH_3$ | O | CH | $-CH_2-CH=CH_2$ | Harz |
| 37 | 4-$CH_3$ | S | N | $-CH_2-CH=CH_2$ | Harz |
| 38 | 4-Cl, 2-$CH_3$ | O | CH | $-CH_2-C\equiv CH$ | Harz |
| 39 | 4-Cl, 2-$CH_3$ | O | CH | -$CH_2$- 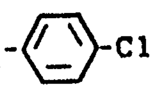 | Harz |

Tabelle 1 (Fortsetzung)

| Beispiel Nr. | $(R^1)_n$ | X | Y | R | Schmelzpunkt [°C] |
|---|---|---|---|---|---|
| 40 | 4-Cl, $2-CH_3$ | O | CH | $-CH_2-$ 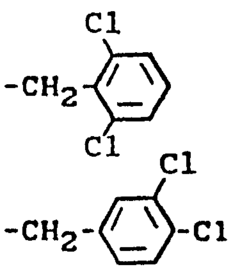 | Harz |
| 41 | 4-Cl, $2-CH_3$ | O | CH | $-CH_2-$ | Harz |
| 42 | 4-Cl, $2-CH_3$ | O | CH | $-CH_2-$ 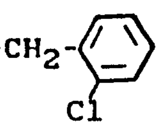 | Harz |
| 43 | 4-Cl | O | N | $-CH_2-$ 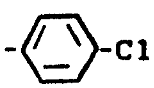 | 73-75 |

In den folgenden Verwendungsbeispielen wurde die nachstehend angegebene Verbindung als Vergleichssubstanz eingesetzt.

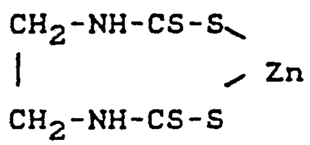 = (A)

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25° C aufgestellt.

4 Tage nach der Inokulation erfolgt die Auswertung des Krankheitsbefalls.

In diesem Test zeigen die erfindungsgemäßen Stoffe, die in den Beispielen 5, 13, 21, 22, 36, 37, 38, 39 und 41 offenbart werden, eine wesentlich bessere Wirksamkeit als die Vergleichssubstanz (A).

Beispiel B

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20° C und 100 % relativer Luftfeuchtigkeit in einer Inkubationskabine.

Die Pflanzen werden in einem Gewächshaus bei einer Temperatur von ca. 15° C und einer relativen Luftfeuchtigkeit von ca. 80 % aufgestellt.

10 Tage nach der Inokulation erfolgt die Auswertung.

In diesem Test zeigen die erfindungsgemäßen Stoffe, die in den Beispielen 5, 2, 8, 11, 27, 28, 29, 30, 36, 38, 39, 40, 41 und 42 offenbart werden, eine wesentlich bessere Wirksamkeit als die Vergleichssubstanz (A).

Beispiel C

Drechslera graminea-Test (Gerste) / Saatgutbehandlung
(syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von $4^{\circ}C$ aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. $18^{\circ}C$ in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

In diesem Test zeigen die erfindungsgemäßen Stoffe, die in den Beispielen 4, 7, 22 und 23 offenbart werden, eine wesentlich bessere Wirksamkeit als die Vergleichssubstanz (A).

## Patentansprüche

1. Substituierte Azolylmethylether der Formel

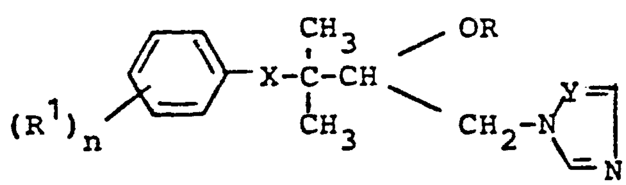

(I)

in welcher

R für Alkyl, Alkenyl, Halogenalkenyl, Alkinyl, Cycloalkyl oder für gegebenenfalls substituiertes Aralkyl steht,

$R^1$ für Halogen, Alkyl, Alkoxy, Alkylthio, Alkylsulfonyl, Halogenalkyl, Halogenalkoxy, Nitro, Cyano, Alkoxycarbonyl oder gegebenenfalls substituiertes Phenyl steht,

X für Sauerstoff oder Schwefel steht,

Y für Stickstoff oder CH steht und

n für die Zahlen 0, 1, 2 oder 3 steht,

sowie deren Säureadditions-Salze und Metallsalz-Komplexe.

2. Substituierte Azolylmethylether der Formel (I), in denen

R für geradkettiges oder verzweigtes Alkyl mit 1 bis 6 Kohlenstoffatomen, Alkenyl mit 2 bis 6 Kohlenstoffatomen, Halogenalkenyl mit 2 bis 6 Kohlenstoffatomen und 1 bis 5 Halogenatomen, Alkinyl mit 2 bis 6 Kohlenstoffatomen, Cycloalkyl mit 3 bis 8 Kohlenstoffatomen oder für gegebenenfalls durch Halogen und/oder Alkyl mit 1 bis 4 Kohlenstoffatomen substituiertes Aralkyl mit 6 bis 10 Kohlenstoffatomen im Arylteil und 1 bis 4 Kohlenstoffatomen im Alkylteil steht,

$R^1$ für Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 gleichen oder verschiedenen Halogenatomen, Halogenalkoxy mit 1 bis 4 Kohlenstoffatomen und 1 bis 5 gleichen

oder verschiedenen Halogenatomen, Nitro, Cyano, Alkoxycarbonyl mit 1 bis 4 Kohlenstoffatomen im Alkoxyteil sowie für gegebenenfalls durch Halogen und/oder Alkyl mit 1 bis 4 Kohlenstoffatomen substituiertes Phenyl steht,

X für Sauerstoff oder Schwefel steht,

Y für Stickstoff oder CH steht und

n für die Zahlen 0, 1, 2 oder 3 steht.

3. Verfahren zur Herstellung von substituierten Azolylmethylethern der Formel

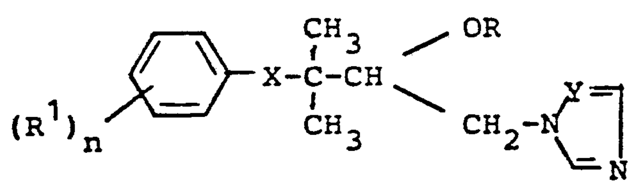

(I)

in welcher

R für Alkyl, Alkenyl, Halogenalkenyl, Alkinyl, Cycloalkyl oder für gegebenenfalls substituiertes Aralkyl steht,

$R^1$ für Halogen, Alkyl, Alkoxy, Alkylthio, Alkylsulfonyl, Halogenalkyl, Halogenalkoxy, Nitro, Cyano, Alkoxycarbonyl oder gegebenenfalls substituiertes Phenyl steht,

X für Sauerstoff oder Schwefel steht,

Y für Stickstoff oder CH steht und

n für die Zahlen 0, 1, 2 oder 3 steht,

sowie von deren Säureadditions-Salzen und Metallsalz-Komplexen, dadurch gekennzeichnet, daß man Azolylmethylcarbinole der Formel

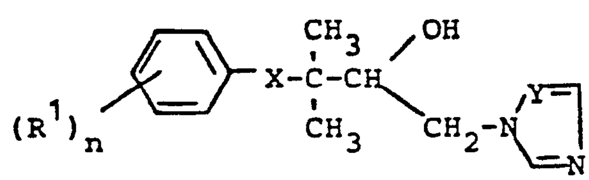

(II)

in welcher

$R^1$, X, Y und n die oben angegebene Bedeutung haben,

mit Verbindungen der Formel

R-Z (III)

in welcher

R die oben angegebene Bedeutung hat

und

Z für Halogen, Tosyl, Mesyl, Sulfat oder eine leicht abspaltbare Säuregruppe steht,

in Gegenwart eines Säurebindemittels sowie in Gegenwart eines Verdünnungsmittels und gegebenenfalls in Gegenwart eines Katalysators umsetzt und gegebenenfalls an die so erhaltenen Verbindungen der Formel (I) eine Säure oder ein Metallsalz addiert.

4. Fungizide Mittel, gekennzeichnet durch einen Gehalt an mindestens einem substituierten Azolylmethylether der Formel (I) bzw. einem Säureadditions-Salz oder Metallsalz-Komplex eines substituierten Azolylmethylethers der Formel (I).

5. Verwendung von substituierten Azolylmethylethern der Formel (I) bzw. von deren Säureadditions-Salzen oder Metallsalz-Komplexen zur Bekämpfung von Pilzen.

6. Verfahren zur Bekämpfung von Pilzen, dadurch gekennzeichnet, daß man substituierte Azolylmethylether der Formel (I) bzw. deren Säureadditions-Salze oder Metallsalz-Komplexe auf Pilze und/oder deren Lebensraum ausbringt.

7. Verfahren zur Herstellung von fungiziden Mitteln, dadurch gekennzeichnet, daß man substituierte Azolylmethylether der Formel (I) bzw. deren Säureadditions-Salze oder Metallsalz-Komplexe mit Streckmitteln und/oder oberflächenaktiven Stoffen vermischt.

8. Substituierter Azolylmethylether der Formel

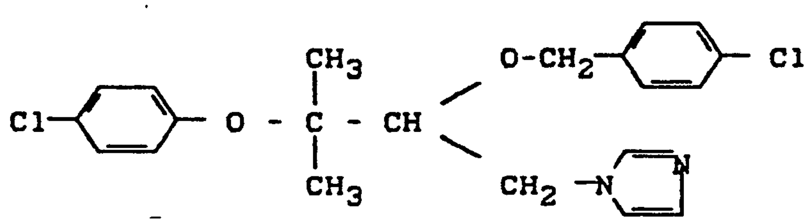

9. Substituierter Azolylmethylether der Formel

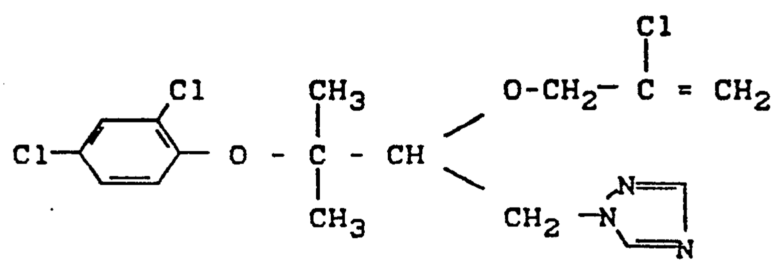

10. Substituierter Azolylmethylether der Formel

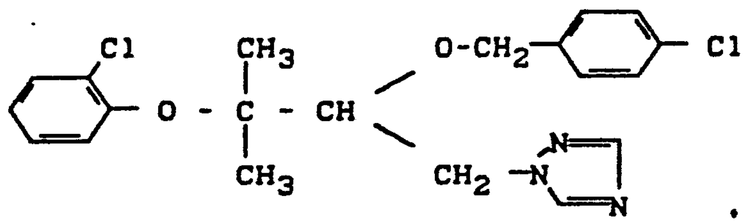

.